# EUROPEAN PATENT APPLICATION

(11) **EP 3 692 958 A1**
(43) Date of publication of application: **12.08.2020**
(21) Application number: 17934413.0
(22) Date of filing: 14.12.2017
(51) Int. Cl.: A61F 13/15, B29C 65/08

(54) **ULTRASONIC SEALING METHOD AND ULTRASONIC SEALING DEVICE**

(30) Priority: 13.12.2017 JP 2017238625
(71) Applicant: Unicharm Corporation, Ehime 799-0111 (JP)
(72) Inventor: NINOMIYA, Akihide, Kanonji-shi, Kagawa 769-1602 (JP); YAMAMOTO, Hiroki, Kanonji-shi, Kagawa 769-1602 (JP); NAKANO, Takumi, Kanonji-shi, Kagawa 769-1602 (JP)
(74) Representative: Staeger & Sperling Partnerschaftsgesellschaft mbB
(86) International application number: PCT/JP2017/044931
(87) International publication number: WO 2019/116507

(57) **Abstract**

An method for ultrasonic-sealing is a method in which a plurality of seal portions (S) are formed in a continuous web (30) at an interval in a direction of transporting the continuous web (30), the continuous web (30) being for an absorbent article, the forming being performed by repeatedly executing ultrasonic-sealing processing on a continuous web (30) that is being transported. The ultrasonic-sealing processing includes: a clamping step of clamping the continuous web (30) with an ultrasonic horn (8) and an anvil (14); a seal-portion forming step of forming the seal portions (S) in the continuous web (30) by vibration of the ultrasonic horn (8) due to receiving an ultrasonic wave generated by an ultrasonic wave generator (9); and an unclamping step of unclamping the continuous web (30). In the seal-portion forming step, the ultrasonic wave generator (9) generates the ultrasonic wave such that a period of time from when the generation of the ultrasonic wave starts until when the generation of the ultrasonic wave ends is longer than a repetition cycle of the ultrasonic-sealing processing.

## Description

### [Technical Field]

The present invention relates to a method and a device for ultrasonic-sealing.

### [Background Art]

There is a well-known method for ultrasonic-sealing in which seal portions are formed at intervals in a direction of transporting a continuous web for an absorbent article by repeatedly executing ultrasonic-sealing processing on the transported continuous web.

This ultrasonic-sealing processing includes: a clamping step in which the continuous web is clamped between an ultrasonic horn and an anvil; a seal-portion forming step in which seal portions are formed in the continuous web by vibration of the ultrasonic horn due to receiving ultrasonic waves emitted by an ultrasonic wave generator; and an unclamping step in which the continuous web is unclamped.

### [Citation List]

### [Patent Literature]

[PTL 1] Japanese Patent Application Publication No. 2002-355270

### [Summary of Invention]

### [Technical Problem]

In order to execute ultrasonic-sealing processing more times and increase the number of absorbent articles produced per unit of time, it is necessary to shorten the repetition cycle of the ultrasonic-sealing processing. However, if the repetition cycle is shortened, the amount of time for forming the seal portions is also shortened, and there is a risk of a decrease in the quality of the seal portions.

The present invention was achieved in light of conventional problems such as that described above, and an aspect of the present invention is to suppress a decrease in the quality of the seal portions.

### [Solution to Problem]

Amain aspect of the present invention for achieving the above-described aspect is an method for ultrasonic-sealing in which a plurality of seal portions are formed in a continuous web at an interval in a direction of transporting the continuous web,
the forming being performed by repeatedly executing ultrasonic-sealing processing on a continuous web that is being transported,
the continuous web being for an absorbent article,
the ultrasonic-sealing processing including:
a clamping step of clamping the continuous web with an ultrasonic horn and an anvil;
a seal-portion forming step of forming the seal portions in the continuous web by vibration of the ultrasonic horn due to receiving an ultrasonic wave generated by an ultrasonic wave generator; and
an unclamping step of unclamping the continuous web,
in the seal-portion forming step,
   the ultrasonic wave generator generating the ultrasonic wave such that a period of time from when the generation of the ultrasonic wave starts until when the generation of the ultrasonic wave ends is longer than a repetition cycle of the ultrasonic-sealing processing.

Features of the present invention other than the above will become clear by reading the description of the present specification with reference to the accompanying drawings.

### [Advantageous Effects of Invention]

According to the present invention, it is possible to suppress a decrease in the quality of the seal portions.

### [Brief Description of the Drawings]

FIG. 1 includes an upper diagram that is a perspective view of an unfolded state of a continuous web 30, and a lower diagram that is a perspective view of the continuous web 30 while being transported to a sealing device 1.
FIG. 2 is a cross-sectional view taken along IX-IX in the lower diagram in FIG. 1, and shows a state where the continuous web 30 is clamped between ultrasonic horns 8 and anvils 14.
FIG. 3 is a cross-sectional view taken along X-X in the lower diagram in FIG. 1 and FIG. 2, and shows a state where the continuous web 30 is clamped between ultrasonic horns 8 and anvils 14.
FIG. 4 is a cross-sectional view taken along I-I in FIG. 9, and shows the sealing device 1 according to an embodiment.
FIG. 5 is a diagram showing a rotating drum 5 of the sealing device 1 and the internal structure thereof.
FIG. 6 is an illustrative diagram for illustrating operations of a swing drive portion.
FIG. 7 is a schematic view of a state in which a sealing unit 10 is clamping the continuous web 30.
FIG. 8 is an enlarged view of the internal structure of a holding portion 52.
FIG. 9 is an illustrative view of operation states of the sealing device 1.
FIG. 10 is an illustrative diagram for illustrating operations of six sealing units 10.
FIG. 11 is an illustrative diagram for illustrating operations of the six sealing units 10 in a low speed mode.
FIG. 12 is an illustrative diagram for illustrating operations of sealing units 10 according to a second embodiment.
FIG. 13 is an illustrative diagram for illustrating operations of sealing units 10 according to a third embodiment.

### [Description of Embodiments]

At least the following matters will become clear with the description of this specification and the attached drawings.

An method for ultrasonic-sealing in which a plurality of seal portions are formed in a continuous web at an interval in a direction of transporting the continuous web,
the forming being performed by repeatedly executing ultrasonic-sealing processing on a continuous web that is being transported,
the continuous web being for an absorbent article,
the ultrasonic-sealing processing including:
a clamping step of clamping the continuous web with an ultrasonic horn and an anvil;
a seal-portion forming step of forming the seal portions in the continuous web by vibration of the ultrasonic horn due to receiving an ultrasonic wave generated by an ultrasonic wave generator; and
an unclamping step of unclamping the continuous web,
in the seal-portion forming step,
   the ultrasonic wave generator generating the ultrasonic wave such that a period of time from when the generation of the ultrasonic wave starts until when the generation of the ultrasonic wave ends is longer than a repetition cycle of the ultrasonic-sealing processing.

According to this method for ultrasonic-sealing, it is possible to ensure sufficient time for appropriately forming the seal portions, and it is possible to suppress a decrease in the quality of the seal portions.

In such a method for ultrasonic-sealing, it is desirable
that the continuous web is transported by rotation of the rotation member with being wound on an outer circumferential surface of a rotation member,
that a plurality of sealing units that each have an ultrasonic horn and an anvil are provided at an equal spacing along a circumferential direction of the rotation member,
that a plurality of first members, which are either of the ultrasonic horns and the anvils, are provided on the outer circumferential surface of the rotation member,
that a plurality of second members, which are other ones out of the ultrasonic horns and the anvils, are each provided on an opposite side of the continuous web so as to be capable of moving toward and away from the corresponding first member,
that the plurality of sealing units sequentially form the plurality of seal portions at the interval while rotating along with rotation of the rotation member, and
that out of the plurality of sealing units, concerning a first sealing unit and a second sealing unit that is adjacent to the first sealing unit in the circumferential direction,
   letting a first period to be a period in which the ultrasonic wave generator for the first sealing unit generates the ultrasonic wave, and a second period to be a period in which the ultrasonic wave generator for the second sealing unit generates the ultrasonic wave, the first period is overlapped with the second period.

According to this method for ultrasonic-sealing, it is possible to ensure sufficient time for appropriately forming the seal portions, and it is possible to suppress a decrease in the quality of the seal portions.

In such a method for ultrasonic-sealing, it is desirable that
the ultrasonic wave generator that generates the ultrasonic wave for the first sealing unit and the ultrasonic wave generator that generates the ultrasonic wave for the second sealing unit are different from each other.

According to this method for ultrasonic-sealing, it is possible to generate optimum ultrasonic waves, and it is possible to further suppress a decrease in the quality of the seal portions.

In such a method for ultrasonic-sealing, it is desirable that
for each of the sealing units, when the sealing unit reaches a predetermined angular position while rotating along with rotation of the rotation member, the ultrasonic wave generator starts to generate the ultrasonic wave.

According to this method for ultrasonic-sealing, ultrasonic waves can be reliably generated at a desired timing regardless of the rotation speed of the rotation member.

In such a method for ultrasonic-sealing, it is desirable that
concerning the first period which is a period in which the ultrasonic wave generator for the first sealing unit generates the ultrasonic wave, and the second period which is a period in which the ultrasonic wave generator for the second sealing unit generates the ultrasonic wave,
for all of the sealing units, the first period is overlapped with the second period.

According to this method for ultrasonic-sealing, it is possible to ensure sufficient time for all of the sealing units to appropriately form the seal portions, and it is possible to suppress a decrease in the quality of the seal portions.

In such a method for ultrasonic-sealing, it is desirable that
concerning the first period which is a period in which the ultrasonic wave generator for the first sealing unit generates the ultrasonic wave, and a third period which is a period in which the ultrasonic wave generator for a third sealing unit that is adjacent to the second sealing unit in the circumferential direction generates the ultrasonic wave,
the first period is not overlapped with the second period.

According to this method for ultrasonic-sealing, it is possible to suppress a sudden change in the load (power) of the device for ultrasonic-sealing.

In such a method for ultrasonic-sealing, it is desirable that
three or more periods of generation of the ultrasonic wave by the ultrasonic wave generator for three or more consecutively adj acent sealing units have a shared overlapping portion.

According to this method for ultrasonic-sealing, it is possible to ensure sufficient time for appropriately forming the seal portions, and it is possible to suppress a decrease in the quality of the seal portions.

In such a method for ultrasonic-sealing, it is desirable
that when the first sealing unit reaches a predetermined clamp angular position while rotating along with rotation of the rotation member,
   the second member comes into contact with the corresponding first member and clamps the continuous web,
that when the first sealing unit reaches a predetermined unclamp angular position while rotating along with rotation of the rotation member,
   the second member moves away from the first member and unclamps the continuous web, and
that in a period from when the first sealing unit starts to clamp the continuous web until when the first sealing unit unclamps the continuous web,
   the ultrasonic wave generator start to generate the ultrasonic wave for the first sealing unit, the second sealing unit, and a third sealing unit that is adjacent to the second sealing unit in the circumferential direction.

According to this method for ultrasonic-sealing, it is possible to ensure sufficient time for appropriately forming the seal portions, and it is possible to suppress a decrease in the quality of the seal portions.

In such a method for ultrasonic-sealing, it is desirable that
concerning the first period which is a period in which the ultrasonic wave generator for the first sealing unit generates the ultrasonic wave, and a third period which is a period in which the ultrasonic wave generator for a third sealing unit that is adjacent to the second sealing unit in the circumferential direction generates the ultrasonic wave,
the first period is not overlapped with the second period.

According to this method for ultrasonic-sealing, it is possible to suppress a sudden change in the load (power) of the device for ultrasonic-sealing.

In such a method for ultrasonic-sealing, it is desirable
that the continuous web is transported by rotation of the rotation member with being wound on an outer circumferential surface of a rotation member,
that a plurality of sealing units that each have an ultrasonic horn and an anvil are provided at an equal interval along a circumferential direction of the rotation member,
that a plurality of first members, which are either of the ultrasonic horns and the anvils, are provided on the outer circumferential surface of the rotation member,
that a plurality of second members, which are other ones out of the ultrasonic horns and the anvils, are each provided on an opposite side of the continuous web so as to be capable of moving toward and away from the corresponding first member,
that the plurality of sealing units sequentially form the plurality of seal portions at the interval while rotating along with rotation of the rotation member,
that the plurality of seal portions are formed in a low speed mode and in a high speed mode,
   the low speed mode being a mode in which the continuous web is transported by rotation of the rotation member at a first speed,
   the high speed mode being a mode in which the continuous web is transported by rotation of the rotation member at a second speed that is higher than the first speed,
out of the plurality of sealing units, concerning a first sealing unit and a second sealing unit that is adjacent to the first sealing unit in the circumferential direction,
   letting a first period to be a period in which the ultrasonic wave generator for the first sealing unit generates the ultrasonic wave, and a second period to be a period in which the ultrasonic wave generator for the second sealing unit generates the ultrasonic wave,
   in the high speed mode, the first period is overlapped with the second period, and
   in the low speed mode, the first period and the second period are not overlapped with each other.

According to this method for ultrasonic-sealing, in both of the modes, it is possible to ensure sufficient time for appropriately forming the seal portions, and it is possible to suppress a decrease in the quality of the seal portions.

An device for ultrasonic-sealing that forms a plurality of seal portions in a continuous web that is for an absorbent article,
the device for ultrasonic-sealing including:
a transporting device configured to transport the continuous web;
an ultrasonic wave generator configured to generate an ultrasonic wave;
an ultrasonic horn configured to form the seal portion in the continuous web that is being transported by the transporting device, by vibrating due to receiving the ultrasonic wave generated by the ultrasonic wave generator; and
an anvil configured to clamp the continuous web together with the ultrasonic horn,
the ultrasonic horn forming the plurality of seal portions at an interval in a direction of transporting the continuous web by repeatedly executing ultrasonic-sealing processing,
   the ultrasonic-sealing processing being for forming a seal portion in the continuous web by vibrating while clamping the continuous web together with the anvil,
the ultrasonic wave generator generating the ultrasonic wave such that a period of time from when the generation of the ultrasonic wave starts until when the generation of the ultrasonic wave ends is longer than a repetition cycle of the ultrasonic-sealing processing.

The above device for ultrasonic-sealing achieves operations and effects similar to those of the method for ultrasonic-sealing described above.

### Method for ultrasonic-sealing of present embodiment

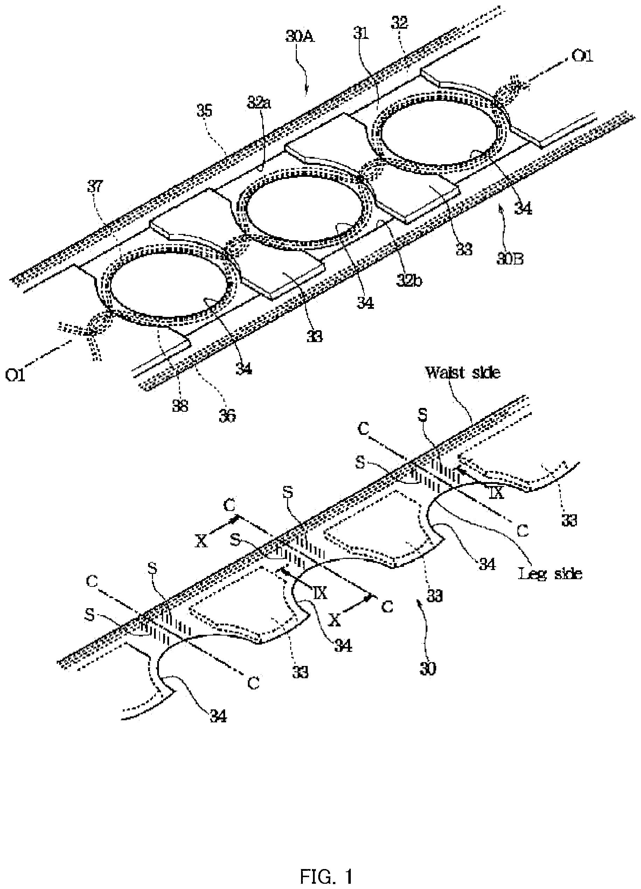

An method for ultrasonic-sealing of the present embodiment is used with an device for ultrasonic-sealing (hereinafter called a sealing device 1). As shown in the lower diagram in FIG. 1, the sealing device 1 is an device for forming multiple seal portions S at intervals in the direction of transporting a continuous web 30 for an absorbent article (the continuous web 30 is a stack of absorbent article base sheets), which is transported along a continuous production line of the absorbent articles, by repeatedly executing ultrasonic-sealing processing on the continuous web 30.

### Continuous web 30 for absorbent article

First, the continuous web 30 for an absorbent article of the present embodiment will be described by way of example of a pull-on disposable diaper.

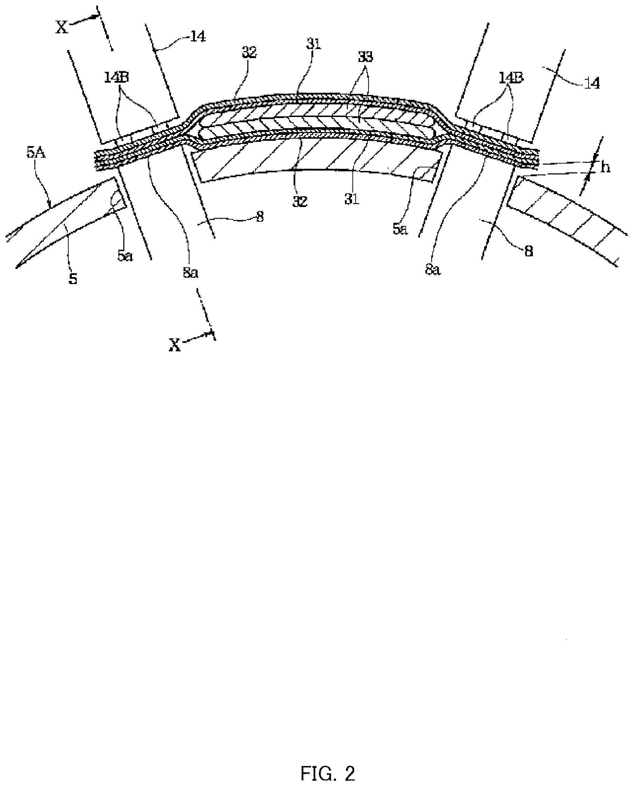

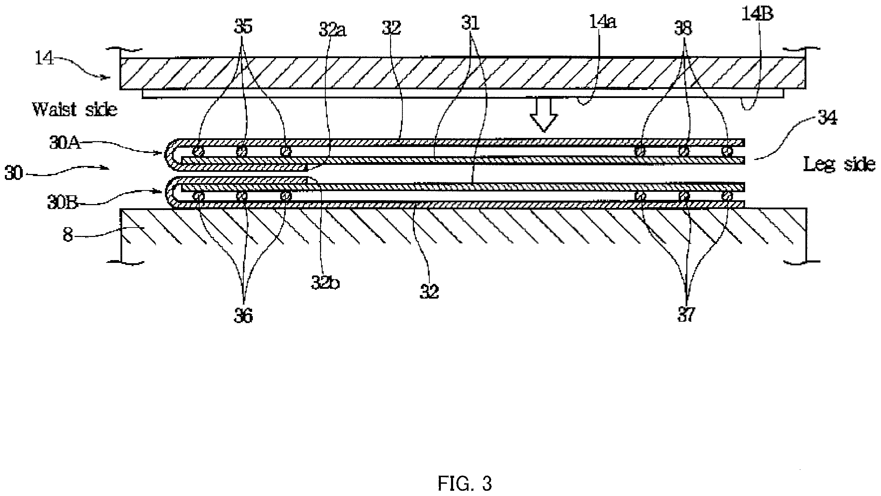

As shown in the lower diagram in FIG. 1, the continuous web 30 of the present embodiment is transported to the sealing device 1 in a folded state. FIG. 2 is cross-sectional view taken along IX-IX in the lower diagram in FIG. 1, and shows a state in which the continuous web 30 is clamped between ultrasonic horns 8 and anvils 14 and seal portions S are being formed in the continuous web 30, and FIG. 3 is a cross-sectional view taken along X-X in the lower diagram in FIG. 1 and FIG. 2.

The lower diagram in FIG. 1 shows a state in which the ultrasonic horns 8 and the anvils 14 of the sealing device 1 have formed seal portions S in the continuous web 30. After the seal portions S have been formed, the continuous web 30 is cut along cut lines C-C between adjacent seal portions S, thus producing pull-on disposable diapers, which are the absorbent article of the present embodiment. Also, the upper diagram in FIG. 1 shows a state in which the continuous web 30 has been unfolded immediately before being transported into the sealing device 1.

As shown in the upper diagram in FIG. 1, when the continuous web 30 is an unfolded belt-like body, a first sheet 32 is located on the underside in the diagram, and a second sheet 31 is stacked thereon. The first sheet 32 is wider than the second sheet 31, and on one side 30A in the upper diagram in FIG. 1, a side edge 32a of the first sheet 32 is folded over onto the second sheet 31. Similarly, on an other side 30B as well, a side edge 32b of the first sheet 32 is folded over onto the second sheet 31. This folded state is shown in FIG. 3.

As indicated by "waist side" in FIG. 3, on the one side 30A of the belt-like body, multiple first waist bands 35 are sandwiched between the first sheet 32 and the second sheet 31. Also, on the other side 30B of the belt-like body, multiple second waist bands 36 are sandwiched between the first sheet 32 and the second sheet 31. The first waist bands 35 and the second waist bands 36 are respectively arranged parallel to each other and extend as straight lines in the direction of transporting the belt-like body.

Also, as indicated by "leg side" in FIG. 3, multiple first leg bands 37 and second leg bands 38 are provided between the first sheet 32 and the second sheet 31. As shown in the upper diagram in FIG. 1, the first leg bands 37 and the second leg bands 38 extend along the direction of transporting the belt-like body while curving in a wave-like shape. Leg holes 34, which form leg insertion portions in the underpants-shaped state, are formed in the regions enclosed within the first leg bands 37 and the second leg bands 38.

The first waist bands 35, the second waist bands 36, the first leg bands 37, and the second leg bands 38 are sandwiched between the first sheet 32 and the second sheet 31 in a state of being stretched by a predetermined factor in the direction of transporting the belt-like body. Also, the first sheet 32 and the second sheet 31 are adhered to the first waist bands 35, the second waist bands 36, the first leg bands 37, and the second leg bands 38, which are sandwiched therebetween, using a hot-melt adhesive or the like.

The first sheet 32 and the second sheet 31 are breathable while also blocking the passage of liquids, and can also be fused together through heat. For example, they can be made of spunbond nonwoven fabric or meltblown nonwoven fabric formed by thermoplastic synthetic fiber, or a laminated body including layers of such nonwoven fabric. Also, a configuration is possible in which either the first sheet 32 or the second sheet 31 is made of nonwoven fabric, and the other one is made of breathable plastic film.

The first waist bands 35, the second waist bands 36, the first leg bands 37, and the second leg bands 38 are elastic stretching members made of rubber or synthetic rubber strings or bands, for example.

A liquid-absorbent body 33 is disposed between adjacent leg holes 34 on the upper surface of the second sheet 31. The liquid-absorbent body 33 is hourglass-shaped or rectangular, and multiple liquid-absorbent bodies 33 are arranged at constant intervals in the direction of transporting the belt-like body. The liquid-absorbent body 33 includes a pulverized pulp, a mixture of a pulverized pulp and a liquid-absorbent polymer (SAP), a stacked body including layers of hydrophilic nonwoven fabric, an air-laid pulp, or the like. These absorbent materials are wrapped in a liquid-permeable top sheet. Each liquid-absorbent body 33 is adhered to the upper surface of the second sheet 31 using a hot-melt adhesive or the like.

The top sheet is formed by spun-lace nonwoven fabric, air-through nonwoven fabric, a plastic film provided with liquid passage holes, or the like.

The belt-like body shown in the upper diagram in FIG. 1 is folded one time onto itself at a center line O1-O1 that extends in the longitudinal direction, thus obtaining the continuous web 30 shown in the lower diagram in FIG. 1. As previously described, the continuous web 30 is transported to the sealing device 1 in the form shown in the lower diagram in FIG. 1. Upon the continuous web 30 being transported to the sealing device 1, the first sheet 32 and the second sheet 31, which are overlaid on each other, are clamped and sealed between the ultrasonic horns 8 and the anvils 14 of the sealing device 1 at locations between adjacent liquid-absorbent bodies 33.

Also, in the present embodiment, as shown in FIG. 3, in intermediate portions of the continuous web 30 that are sandwiched between the ultrasonic horns 8 and the anvils 14, four sheets altogether, specifically two portions of the first sheet 32 and two portions of the second sheet 31, are overlaid on each other. The thickness of the continuous web 30 is the smallest in the intermediate portions. On the waist side, in addition to the four sheets, the two side edges of the first sheet 32 are folded back, and the first waist bands 35 and the second waist bands 36 are also sandwiched between the sheets, thus forming the thickest portions. On the leg side corresponding to the leg hole 34 side, the four sheets and the first leg bands 37 and the second leg bands 38 arranged therebetween are provided, and the thickness on the leg side is higher than in the intermediate portions, but lower than on the waist side.

The first sheet 32 and the second sheet 31 are formed from a heat-fusing material, and therefore when heat is generated therein by vibration applied by the ultrasonic horn 8, the sheets become fused together in accordance with a micro protrusion pattern that is formed on an anvil facing surface 14a of the anvil 14, thus forming seal portions S.

In the example shown in the lower diagram in FIG. 1, the pattern of the seal portions S formed by the micro protrusion pattern is a column of thin seal lines. After the seal portions S have been formed by the sealing device 1, the continuous web 30 is cut along cut lines C-C located between adjacent seal portions S, thus forming pull-on disposable diapers corresponding to the absorbent article.

Note that although the manufacturing of a pull-on disposable diaper has been described above as an example of the manufacturing of an absorbent article, the absorbent article manufactured by the sealing device 1 of the present invention may be a sanitary napkin, a panty liner, or the like.

### Sealing device 1

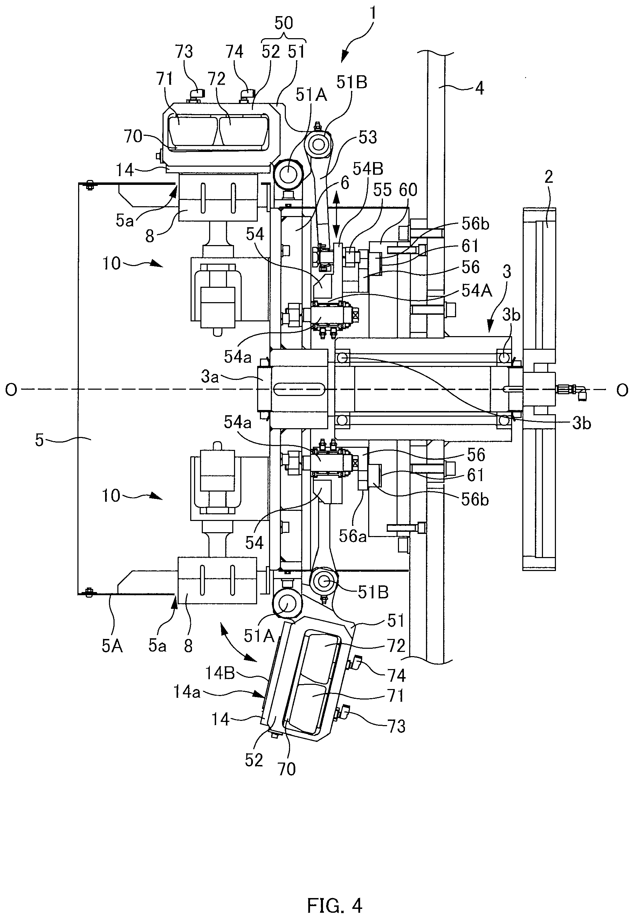

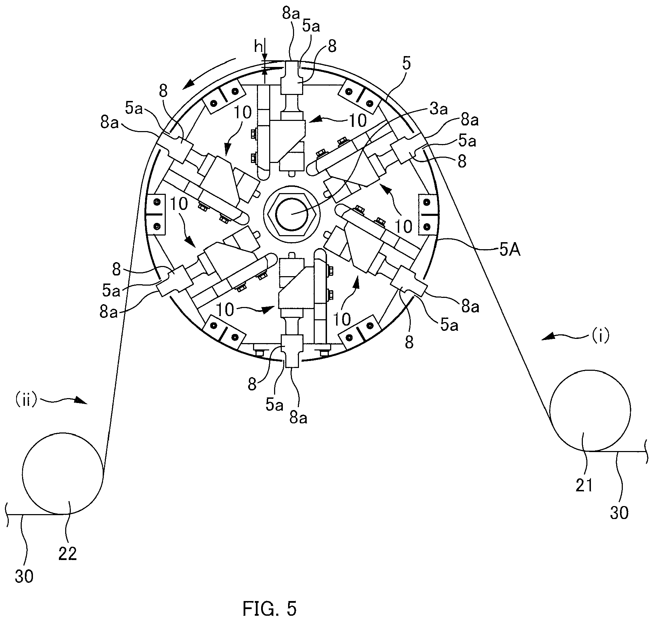

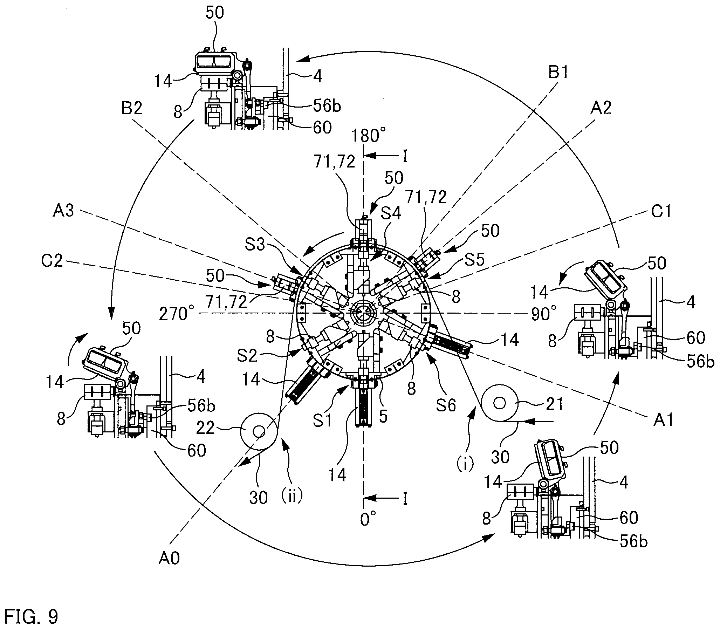

Next, the sealing device 1 will be described. FIG. 4 is a cross-sectional view of the sealing device 1 according to the present embodiment (a cross-section along line I-I in FIG. 9). FIG. 5 is a diagram showing a rotating drum 5 of the sealing device 1 and the internal structure thereof.

The sealing device 1 includes a rotation-member drive portion, a rotation member (corresponding to a transporting device), sealing units 10, ultrasonic wave generators 9, swinging support members 50, and a swing drive portion.

As shown in FIG. 4, the rotation-member drive portion includes a rotation shaft 3a, a bearing portion 3, ball bearings 3b, a timing wheel 2, a toothed belt (not shown), and a motor (not shown). Also, the bearing portion 3 is provided on a fixed table 4, which is a fixed portion, and the rotation shaft 3a is rotatably supported by the ball bearings 3b that are held in the bearing portion 3. In FIG. 4, the center line of the rotation shaft 3a is indicated by a rotation center line 0-0.

The timing wheel 2, which has teeth on its outer circumferential surface, is fixed to the base end portion of the rotation shaft 3a on the right side in FIG. 4, and the toothed belt is hung around the timing wheel 2. When a drive source that has a motor applies motive power to the timing wheel 2 via the toothed belt, the rotation shaft 3a continuously rotates in the counter-clockwise direction as seen from the left side in FIG. 4.

The rotation member is for transporting the continuous web 30, and includes a rotation base 6 and the rotating drum 5 as shown in FIG. 4. The rotation member is fixed to the rotation shaft 3a, and rotates due to the rotation-member drive portion. Specifically, the rotation base 6 is fixed to the rotation shaft 3a, the rotating drum 5 is fixed to the rotation base 6, and these members rotate along with the rotation shaft 3a. The rotation base 6 is parallel with and opposes the fixed table 4 across a gap for ensuring space for provision of the swinging support members 50 and the swing drive portion.

As shown in FIGS. 4 and 5, an outer circumferential surface 5A of the rotating drum 5 is provided with multiple windows 5a that are rectangular and extend parallel with the rotation center line O-O. The windows 5a are formed at an even pitch in the circumferential direction of the rotating drum 5, and in the present embodiment, six windows 5a are provided at an arrangement angle of 60 degrees about the rotation center line O-O.

The sealing units 10 each include an ultrasonic horn 8, a booster 7b, a converter 7a, and an anvil 14, and are provided on the rotation member.

Accordingly, the sealing units 10 rotate along with the rotation base 6 and the rotating drum 5. In the present embodiment, as shown in FIG. 5, multiple (six) sealing units 10 are provided at an equiangular arrangement angle of 60 degrees along the circumferential direction of the rotation member. Hereinafter, the sealing units 10 will be called a sealing unit S1, a sealing unit S2, a sealing unit S3, a sealing unit S4, a sealing unit S5, and a sealing unit S6, in order along the circumferential direction.

As shown in FIG. 4, the ultrasonic horn 8 is fixed to the rotation base 6 at a location inside the rotating drum 5. The ultrasonic horns 8 and the devices connected to the ultrasonic horns 8 are arranged in a radiating manner about the rotation center line O-O, and the arrangement angle thereof matches the arrangement angle of the windows 5a. Also, as shown in FIG. 5, the ultrasonic horns 8 each protrude outward from the corresponding window 5a of the rotating drum 5 to a projecting height h, a horn facing surface 8a at the leading end of the ultrasonic horn 8 faces outward in the normal direction (radial direction) from the rotation center line O-O, and the horn facing surface 8a is parallel with the rotation center line O-O.

The anvils 14 are fixed to support portions 70 of holding portions 52 of the swinging support members 50, and move toward and away from the horn facing surfaces 8a as the swinging support members 50 swing. In other words, the anvils 14 each move to a position in contact with the corresponding ultrasonic horn 8, and move to a distant position rotated about 90 degrees.

Note that the sealing units 10 will be described in further detail later.

As shown in FIG. 4, each of the swinging support members 50 is constituted by a swing portion 51 and a holding portion 52, and the swing portion 51 and the holding portion 52 are fixed. The anvils 14 are fixed to the support portions 70, which are provided on the holding portions 52. The swinging support members 50 are provided on the rotation member. Accordingly, the swinging support members 50 rotate along with the rotation base 6 and the rotating drum 5. Note that the holding portions 52 will be described in further detail later.

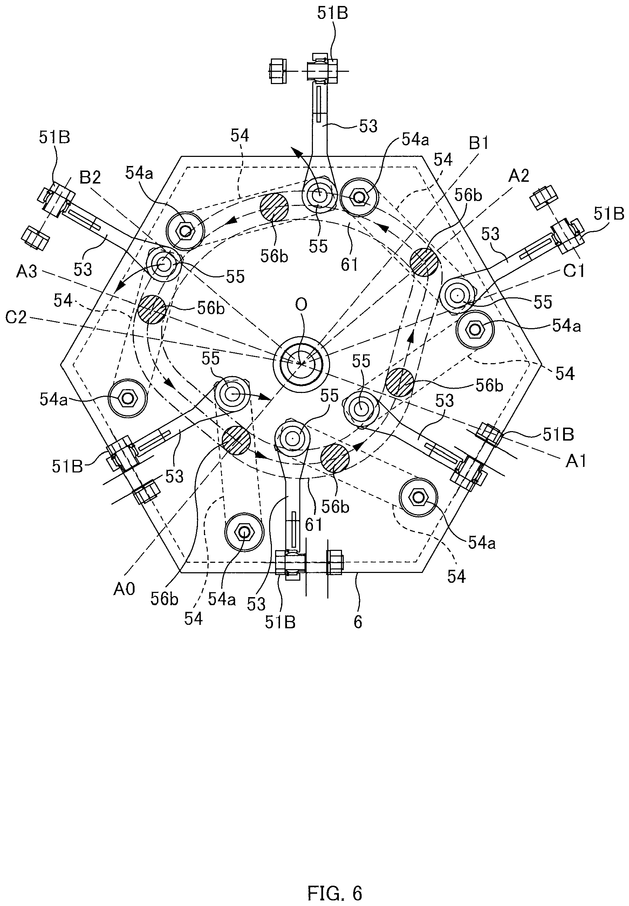

FIG. 6 is an illustrative diagram for illustrating swinging operations of the swinging support members 50. As shown in FIG. 6, the rotation base 6 is has a regular hexagonal front surface. The six swinging support members 50 are provided at intervals of 60 degrees at the center portions of the sides of the regular hexagon, and the arrangement angle of the swinging support members 50 matches the arrangement angle of the ultrasonic horns 8.

As shown in FIG. 4, the swing portions 51 are swingably supported to respective sides of the regular hexagon of the rotation base 6 by swing shafts 51A, and the swing shafts 51A are oriented orthogonal to the rotation center line O-O. Accordingly, the swinging support members 50 (holding portions 52) can swing the anvils 14 about the swing shafts 51A to a position radiating away from rotation center line O-O.

As shown in FIG. 4, in the swing drive portion, a cam member 60 that constitutes the swing drive portion is fixed to the upper surface of the fixed table 4 that faces the rotation base 6. The cam member 60 is shaped as a flat plate that has a predetermined thickness, and as shown in FIG. 6, the front surface is provided with a cam groove 61 that serves as a cam track. The cam groove 61 is continuous around the rotation center line O-O. Also, the recession depth direction of the cam groove 61 is parallel with the rotation center line O-O.

As shown in FIGS. 4 and 6, a link mechanism is provided between the cam member 60 and each of the swing portions 51. In the link mechanism, drive links 53 are rotatably supported to the swing portions 51 via first coupling shafts 51B that are oriented orthogonal to the rotation center line 0-0.

Base portions 54A of rotation links 54 are rotatably supported to the rear surface of the rotation base 6 by support shafts 54a. The axial directions of the support shafts 54a are parallel with the rotation center line 0-0. The leading end portions of the drive links 53 are rotatably coupled to the leading end portions of arm portions 54B of the rotation links 54 via second coupling shafts 55. The axial directions of the second coupling shafts 55 are parallel with the rotation center line 0-0.

A drive member 56 is attached to each of the rotation links 54. The drive member 56 has a drive support body 56a that is non-rotatably fixed to an intermediate portion of the rotation link 54, and a follower 56b that is provided on the drive support body 56a. The follower 56b can move inside the cam groove 61. The follower 56b rolls inside the cam groove 61, or slides therein without rolling.

Note that FIG. 6 shows the relative positional relationship that the rotation links 54 and the followers 56b have with the drive links 53, and the drive support bodies 56a are not shown.

As shown in FIG. 6, when the rotation base 6 rotates, the followers 56b move along the cam groove 61. At this time, the distance between each of the followers 56b and the rotation center line 0-0 changes according to the shape of the cam groove 61. The rotation links 54 rotate in accordance with this change, and the swinging support members 50 also rotate via the drive links 53.

As previously described, the swinging support members 50 rotate due to the cam groove 61, and as a sealing unit 10 moves from an angular position A2 to an angular position A3 shown in FIG. 6, the swinging support member 50 rotates toward the outer circumferential surface 5A of the rotating drum 5 and then reaches a contact position, as shown by the sealing unit 10 at the top in FIG. 4. Also, as the sealing unit 10 moves from an angular position A0 to an angular position A1 shown in FIG. 6, the swinging support member 50 rotates by about 90 degrees toward the fixed table 4 to a retracted position (see FIG. 9), as shown by the sealing unit 10 at the bottom in FIG. 4.

### Sealing units 10 and ultrasonic wave generators 9

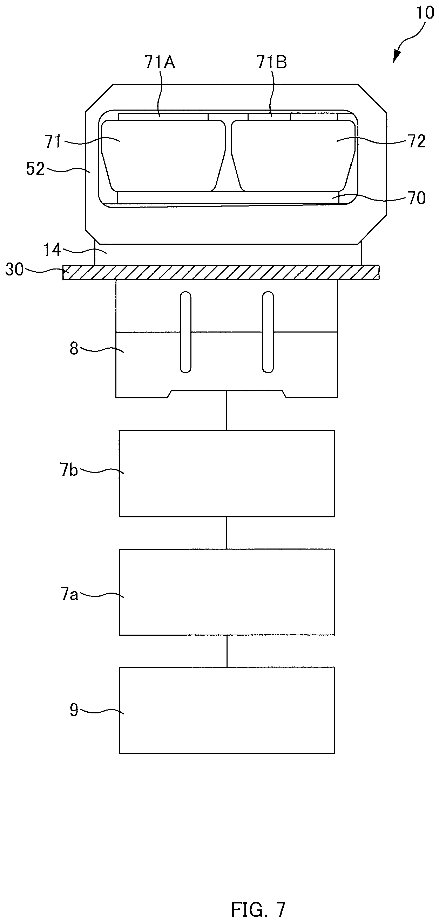

FIG. 7 is a schematic view of a state in which one of the sealing units 10 is clamping the continuous web 30.

In the sealing unit 10, the transported continuous web 30 is clamped between the ultrasonic horn 8 and the anvil 14, an ultrasonic signal emitted (generated) by the ultrasonic wave generator 9 is converted into mechanical ultrasonic vibration by the converter 7a, and the ultrasonic horn 8 receives the mechanical ultrasonic vibration via the booster 7b. The ultrasonic horn 8 vibrates upon receiving the mechanical ultrasonic vibration, and a seal portion S is thus formed in the continuous web 30.

In the present embodiment, a different ultrasonic wave generator 9 generates ultrasonic waves for each of the sealing units 10. In other words, one ultrasonic wave generator 9 is provided for each of the sealing units 10. Accordingly, as shown in FIG. 7, each sealing unit 10 has one ultrasonic horn 8, one booster 7b, one converter 7a, and one anvil 14, and is connected to one ultrasonic wave generator 9. Note that the ultrasonic wave generators 9 are provided outside of the rotation member and are connected thereto via a slip ring (not shown) . Also, first members, which are either the ultrasonic horns 8 or the anvils 14 (in the present embodiment, the ultrasonic horns 8), are provided on the outer circumferential surface 5A of the rotation member (rotating drum 5), and second members, which are the other ones (in the present embodiment, the anvils 14), are provided on the opposite side of the continuous web 30 so as to be able to move toward and away from the first members (ultrasonic horns 8).

The ultrasonic wave generator 9 is a device that emits ultrasonic waves (an ultrasound electrical signal) . The frequency of the ultrasonic waves emitted by the ultrasonic wave generator 9 (hereinafter, also called the ultrasonic frequency) is equivalent to the natural frequency of the ultrasonic horn 8 (i.e., the resonance frequency, which in stricter terms is the natural frequency of the ultrasonic stack including the converter 7a and the booster 7b as well, but will be described here as the natural frequency of the ultrasonic horn 8) . In other words, the size (length) of the ultrasonic horn 8 is designed such that the ultrasonic frequency emitted by the ultrasonic wave generator 9 (in the present embodiment, 20kHz) is equivalent to the natural frequency of the ultrasonic horn 8.

The converter 7a is connected to the ultrasonic wave generator 9, and converts the ultrasound electrical signal emitted by the ultrasonic wave generator 9 into mechanical vibration. The converter 7a is provided with a piezoelectric element, and the aforementioned conversion is performed by the piezoelectric element.

The booster 7b is connected to the converter 7a, and amplifies the vibration received from the converter 7a.

The ultrasonic horn 8 is connected to the booster 7b. And, the ultrasonic horn 8 generates frictional heat at the interface between overlaid base materials in the continuous web 30 by vibrating due to receiving ultrasonic waves emitted by the ultrasonic wave generator 9 (ultrasonic waves in the form of mechanical vibration due to the conversion into mechanical vibration performed by the converter 7a), that is to say by applying mechanical energy from vibration to the continuous web 30. Accordingly, the base materials melt and form a seal portion S in the continuous web 30.

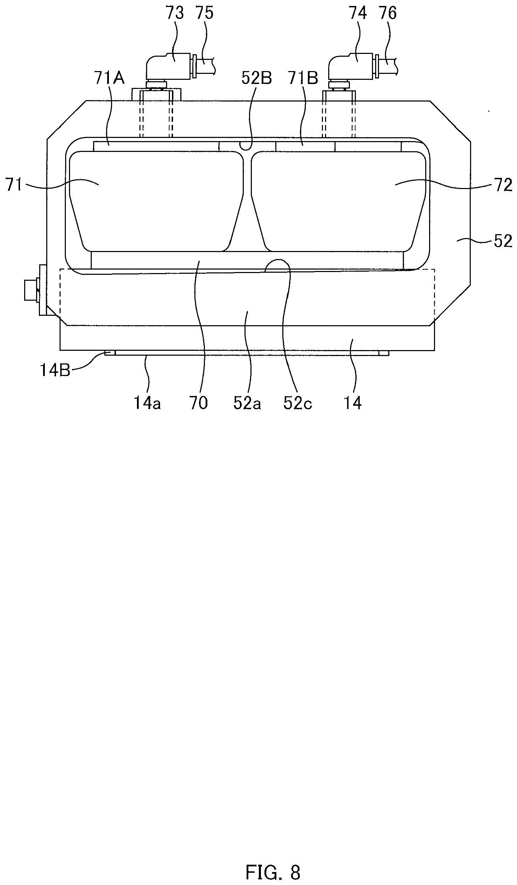

The anvil 14 is a member for performing sealing by clamping the continuous web 30 together with the ultrasonic horn 8. The surface of the anvil 14 is provided with projection portions 14B (see FIG. 8), and the surfaces of the projection portions 14b make up the anvil facing surface 14a that faces the ultrasonic horn 8. The anvil facing surface 14a is a patterned surface set with a pattern corresponding to the seal that is to be formed in the continuous web 30. The anvil 14 is fixed to a support portion 70, which is provided inside the holding portion 52, so that the patterned surface faces outward from the holding portion 52, and the support portion 70 supports a first elastic member 71 and a second elastic member 72 that will be described later.

### Holding portion 52

FIG. 8 is an enlarged view of the internal structure of one holding portion 52. The holding portion 52 includes the first elastic member 71 and the second elastic member 72, and the anvil 14 is fixed to the support portion 70 that supports the first elastic member 71 and the second elastic member 72. The first elastic member 71 and the second elastic member 72 are arranged side-by-side in the width direction of the rotating drum 5, that is to say in a direction along the rotation center line 0-0. The holding portion 52 swings due to the swing drive portion so as to move the anvil 14 toward or away from the ultrasonic horn 8. The anvil 14 and the ultrasonic horn 8 clamp the continuous web 30 when the holding portion 52 is at a contact position at which the anvil 14 and the ultrasonic horn 8 are in contact with each other.

The first elastic member 71 and the second elastic member 72 are each an air damper or an air spring that includes a bag body (casing) that is flexibly deformable and elastically deformable and made of an elastically deformable material such as rubber or rubber embedded with a reinforcing material. The hollow portion of the bag body receives a supply of air as a fluid such that the interior is set to a predetermined pressure.

As shown in FIG. 8, the attachment sides of the first elastic member 71 and the second elastic member 72 are fixed to an attachment surface 52B of the holding portion 52 via a first fixing plate 71A and a second fixing plate 71B. The support portion 70 is fixed to the pressure application sides of the first elastic member 71 and the second elastic member 72, and the anvil 14 is fixed to the support portion 70. When the anvil 14 is not in contact with the ultrasonic horn 8, the support portion 70 is pressed against an inward surface 52c of a frame portion 52a due to the fluid pressure in the first elastic member 71 and the second elastic member 72.

The first fixing plate 71A and the second fixing plate 71B are respectively provided with a first nozzle 73 and a second nozzle 74, and the first nozzle 73 and the second nozzle 74 are respectively connected to a first air pipe 75 and a second air pipe 76. In other words, the first elastic member 71 and the second elastic member 72 each have an air supply portion, and the internal pressures thereof can be set individually.

As previously described, the continuous web 30 of the present embodiment is the above-described continuous body of pull-on disposable diapers, and as shown in FIG. 3, the continuous web 30 is not flat in the portions where the seal portions S are formed by the ultrasonic horns 8 and the anvils 14. In other words, the thickness is highest on the waist side, the thickness is lower in the intermediate portion, and the thickness is then higher on the leg side. Accordingly, the continuous web 30 that is to be subjected to sealing has different thicknesses in different portions, and the surface has an uneven shape.

Even in the case where the thickness is not even at the locations where the seal portions S are to be formed, and instead is different at different locations as with a disposable diaper, the anvil facing surface 14a can flexibly accommodate changes in the thickness of the continuous web 30 because the anvil 14 is pressed against the ultrasonic horn 8 by the air dampers that are bag-shaped elastic bodies filled with air (a fluid). In other words, due to the pressure inside the first elastic member 71 and the second elastic member 72, the ultrasonic horn 8 and the anvil 14 can substantially evenly press the locations where the seal portions S are to be formed in the continuous web 30. Accordingly, the seal quality can be made uniform for the seal portion S locations.

Also, the pressure inside the bag bodies of the first elastic member 71 and the second elastic member 72 can be controlled by the supply of air from the first nozzle 73 and the second nozzle 74. Accordingly, control for changing the pressure inside the bag bodies can be easily performed in accordance with the material and the structure of the continuous web 30 that is to be subjected to sealing. For this reason, even if the structure of the continuous web 30 is changed, and the sealing pattern of the anvil facing surface 14a is accordingly changed, simply changing the pressure inside the bag bodies makes it possible to complete the setting for that stage, and to always perform sealing with optimal conditions.

### Operations of sealing device 1

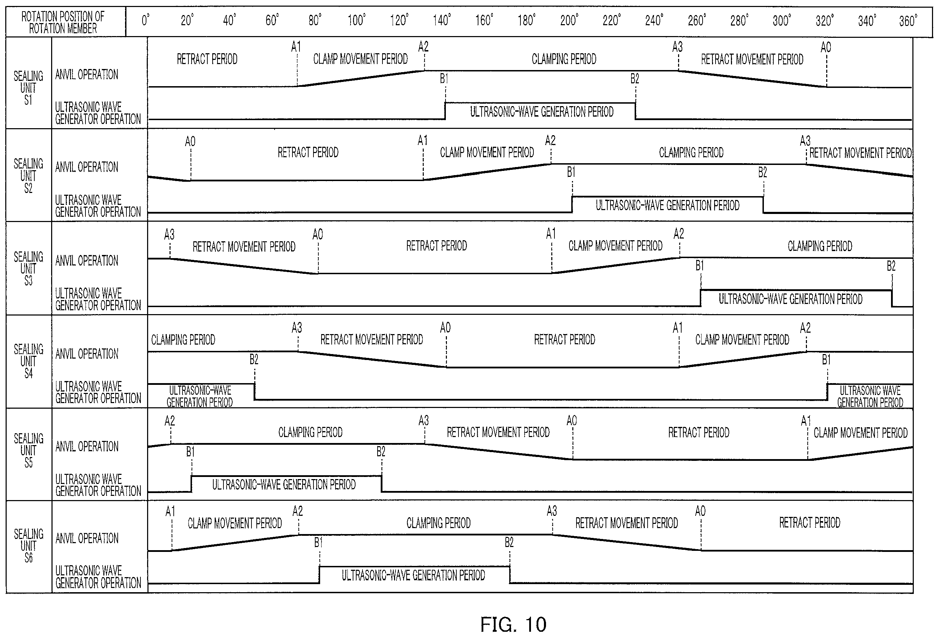

Next, operations of the sealing device 1 will be described with reference to FIGS. 1 to 6 and 9 to 10. FIG. 9 is an illustrative view of operation states of the sealing device 1. FIG. 10 will be described later.

As shown in FIG. 5, the continuous web 30 is wound on a supply transporting roll 21 and transported to the outer circumferential surface 5A of the rotating drum 5. The continuous web 30 is wound on the outer circumferential surface 5A of the rotating drum 5 (more specifically, the horn facing surfaces 8a of the ultrasonic horns 8 that project from the outer circumferential surface 5A) over an angle of about 180 degrees, and then separates from the rotating drum 5 in a discharge section (ii) , is wound around a discharge transporting roll 22, and is drawn to the outside.

The continuous web 30 is continuously fed to the supply section (i) at a constant speed, and in the sealing device 1, rotation motive power is transmitted to the timing wheel 2, and the rotation shaft 3a as well as the rotating drum 5 and the rotation base 6 (which constitute the rotation member) rotate at a constant angular speed in the counter-clockwise direction in FIGS. 5 and 9.

Here, the continuous web 30 comes into contact with the horn facing surfaces 8a of the ultrasonic horns 8 that project from the outer circumferential surface 5A of the rotating drum 5, and the rotating drum 5 rotates in this state. In the present embodiment, the angular speed of the rotation member is set such that the rotation circumferential speed of the horn facing surfaces 8a matches the supply speed of the continuous web 30. Accordingly, on the outer circumferential surface 5A of the rotating drum 5, the horn facing surfaces 8a of the ultrasonic horns 8 and the continuous web 30 move together without sliding relative to each other. In this way, the continuous web 30 is transported due to rotation of the rotation member (rotating drum 5) while the continuous web 30 is wound on the outer circumferential surface 5A of the rotation member (rotating drum 5).

Also, the circumferential arrangement pitch of the ultrasonic horns 8 that project from the outer circumferential surface 5A of the rotating drum 5 matches the arrangement pitch of the liquid-absorbent bodies 33 and the arrangement pitch of the leg holes 34 in the continuous web 30 shown in the lower diagram in FIG. 1. Accordingly, when the continuous web 30 is transported to the outer circumferential surface 5A of the rotating drum 5, as shown in FIG. 2, each liquid-absorbent body 33 is located between a pair of adjacent ultrasonic horns 8, and portions not including the liquid-absorbent body 33 are arranged on the horn facing surfaces 8a of the ultrasonic horns 8.

While the rotation base 6 and the rotating drum 5 rotate in the counter-clockwise direction, the followers 56b provided in the swing drive portion move along the cam groove 61 of the cam member 60 provided on the fixed table 4.

As shown in FIGS. 6 and 9, as each sealing unit 10 (the anvil 14 and the ultrasonic horn 8) moves from the predetermined angular position A0 to the predetermined angular position A1 due to the rotation of the rotation base 6, the corresponding follower 56b moves toward the rotation center line O-O due to the cam groove 61. Accordingly, at this time, as shown by the sealing unit 10 at the bottom in FIG. 4, the corresponding swinging support member 50 rotates about the swing shaft 51A so as to face outward in the radiating direction, and the anvil 14 held by the swinging support member 50 faces outward at an angle of about 90 degrees relative to the rotation center line O-O.

When the sealing unit 10 (the anvil 14 and the ultrasonic horn 8) moves from the angular position A1 to the predetermined angular position A2 (corresponding to a predetermined clamp angular position) , the follower 56b is guided by the cam groove 61 so as to move circumferentially outward. Accordingly, the swinging support member 50 rotates toward the outer circumferential surface 5A of the rotating drum 5, and when the angular position A2 is reached, the anvil 14 and the ultrasonic horn 8 clamp the continuous web 30 (the anvil 14 comes into contact with the ultrasonic horn 8, and clamps the continuous web 30, and this corresponds to a clamping step) . Specifically, as shown in FIG. 2, in the region of the continuous web 30 in which the liquid-absorbent body 33 is not provided, the stacked body, which includes the first sheet 32, the second sheet 31, the first waist bands 35, the second waist bands 36, the first leg bands 37, and the second leg bands 38, is compressed between the horn facing surface 8a of the ultrasonic horn 8 and the anvil facing surface 14a of the anvil 14.

Note that as shown in FIG. 9, when the sealing unit 10 (the anvil 14 and the ultrasonic horn 8) reaches a predetermined angular position C1 that is along the path from the angular position A1 to the angular position A2, the ultrasonic horn 8 moves from a separated state of being separated from the continuous web 30 to a contact state of being in contact with the continuous web 30.

This clamping state continues until the predetermined angular position A3 (corresponding to a predetermined unclamp angular position) is reached, and during that time (i.e., while the continuous web 30 is clamped by the anvil 14 and the ultrasonic horn 8 from the angular position A2 to the angular position A3), the seal portions S are formed. In other words, the ultrasonic-sealing processing for forming the seal portions S is processing in which the ultrasonic horn 8 and the anvil 14 clamp the continuous web 30, the seal portions S are formed in the continuous web 30 by vibration of the ultrasonic horn 8 due to receiving ultrasonic waves emitted by the ultrasonic wave generator 9, and then the continuous web 30 is unclamped. This ultrasonic-sealing processing is executed in the period of time from the angular position A2 to the angular position A3.

When the sealing unit 10 (the anvil 14 and the ultrasonic horn 8) reaches a predetermined angular position B1, the ultrasonic wave generator 9 starts to emit ultrasonic waves for the ultrasonic horn 8. Note that in the present embodiment, the angular position of the sealing unit 10 can be detected by an encoder, and an electrical signal is used to notify the ultrasonic wave generator 9 that the sealing unit 10 (the anvil 14 and the ultrasonic horn 8) has reached the angular position B1. The ultrasonic horn 8 vibrates upon receiving ultrasonic waves from the ultrasonic wave generator 9, and forms a seal portion S in the continuous web 30 (corresponding to a seal-portion forming step).

After the ultrasonic wave generator 9 starts to emit ultrasonic waves, when the ultrasonic vibration energy (which corresponds to the electric energy of the ultrasonic wave generator 9) reaches a predetermined amount, the ultrasonic wave generator 9 stops the ultrasonic waves (stops generating ultrasonic waves) . The predetermined amount is determined in advance in consideration of appropriate formation of the seal portion S. Also, the predetermined amount is determined such that the generation of ultrasonic waves is stopped before the sealing unit 10 (the anvil 14 and the ultrasonic horn 8) reaches the angular position A3 (in the present embodiment, as shown in FIG. 9, the ultrasonic wave generator 9 stops generating ultrasonic waves at the angular position B2).

When the sealing unit 10 (the anvil 14 and the ultrasonic horn 8) reaches the angular position A3, the anvil 14 and the ultrasonic horn 8 unclamp the continuous web 30 (the anvil 14 moves away from the ultrasonic horn 8, thus unclamping the continuous web; corresponding to an unclamping step) . Then, as the sealing unit 10 (the anvil 14 and the ultrasonic horn 8) moves from the angular position A3 to the angular position A0, the follower 56b is guided toward the rotation center line 0-0 by the cam groove 61, and the anvil 14 rotates away from the ultrasonic horn 8 and the continuous web 30. When the angular position A0 is reached, as shown by the sealing unit 10 at the bottom in FIG. 4 again, the anvil 14 rotates to a retracted position that is at an angle of about 90 degrees relative to the rotation center line O-O.

Note that as shown in FIG. 9, when the sealing unit 10 (the anvil 14 and the ultrasonic horn 8) reaches a predetermined angular position C2 that is along the path from the angular position A3 to the angular position A0, the ultrasonic horn 8 moves from a contact state of being in contact with the continuous web 30 to a separated state of being separated from the continuous web 30. After separating from the ultrasonic horn 8, the continuous web 30 is transported out of the sealing device 1 by the discharge transporting roll 22, and is cut at the cut lines C-C shown in the lower diagram in FIG. 1, thus producing individual pull-on disposable diapers.

### Operations of six sealing units 10

Whereas the above description of operations is focused on one of the six sealing units 10 provided in the sealing device 1, the following describes how the six sealing units 10 operate, that is to say the relationship between the operations of the six sealing units 10, with reference to FIG. 10. FIG. 10 is an illustrative diagram for illustrating operations of the six sealing units 10.

The six sealing units 10 all perform the previously described operations. In other words, when the sealing units 10 reach the previously described angular positions A0, A1, A2, A3, B1, and B2, they perform the previously described operations that correspond to those angular positions (note that as previously described, the generation of ultrasonic waves is stopped when the ultrasonic vibration energy reaches the predetermined amount, and therefore the ultrasonic waves do not necessarily stop when the angular position B2 is reached, but rather there is a certain amount of variation. However, in the present embodiment, for the sake of convenience, the following description is based on the presumption that the ultrasonic waves have stopped at the angular position B2 for all of the six sealing units 10).

However, as previously described, the six sealing units 10 are provided at equal intervals corresponding to an arrangement angle of 60 degrees along the circumferential direction of the rotation member that rotates, and therefore there is a time difference between when the sealing units 10 reach each of the angular positions. Accordingly, there is also a difference between the operation timings of the operations of the sealing units 10.

The top section in FIG. 10 shows rotation positions of the rotation member (the rotation base 6 and the rotating drum 5). In the present embodiment, the rotation positions of the rotation member are considered to be the same as the angular positions of the sealing unit S1, which is one of the six sealing unit 10. In other words, when the rotation member is located at the 0 degree rotation position, the sealing unit S1 is also located at the 0 degree angular position.

The sections below the "rotation position of rotation member" field show operations performed by the sealing unit S1, the sealing unit S2, the sealing unit S3, the sealing unit S4, the sealing unit S5, and the sealing unit S6 in correspondence with the rotation positions. For each sealing unit, these operations are divided into operations of the anvil 14 and operations of the ultrasonic wave generator 9.

As previously described, each sealing unit 10 operates in the sequence of: start of movement for clamping <angular position A1> → clamping <angular position A2> → ultrasonic wave generation <angular position B1> → stop of ultrasonic waves <angular position B2> → unclamping (start of movement for retraction) <angular position A3> → retraction <angular position A0> → and so on; and as shown in the row for "sealing unit S1", these operations are respectively executed when the rotation position of the rotation member reaches about 70 degrees, about 130 degrees, about 140 degrees, about 230 degrees, about 250 degrees, and about 320 degrees. Note that because the rotation positions of the rotation member and the angular positions of the sealing unit S1 match each other, the sealing unit S1 is also located at the angular positions of about 70 degrees, about 130 degrees, about 140 degrees, about 230 degrees, about 250 degrees, and about 320 degrees when performing the aforementioned operations.

Also, the sealing unit S2 is the sealing unit 10 that is adjacent to the sealing unit S1 in the circumferential direction, and is separated by an angle of 60 degrees from the sealing unit S1. Accordingly, the sealing unit S2 performs the same operations as the sealing unit S1, at a delay of 60 degrees from the sealing unit S1. Accordingly, the operation graph in the row for "sealing unit S2" corresponds to a 60-degree shift rightward from the operation graph in the row for "sealing unit S1",and the above-described operations are executed when the rotation position of the rotation member reaches angles obtained by adding 60 degrees to the angles for the sealing unit S1, that is to say about 130 degrees, about 190 degrees, about 200 degrees, about 290 degrees, about 310 degrees, and about 20 degrees. Note that for these operations, the sealing unit S2 is also located at the angular positions of about 70 degrees, about 130 degrees, about 140 degrees, about 230 degrees, about 250 degrees, and about 320 degrees, and this is similar to the case of the sealing unit S1 (i.e., the rotation positions of the rotation member are different for the operations, but the angular positions of the sealing unit 10 are the same for the operations).

Hereinafter, operations are similarly executed in the case of the sealing units S3 to S6 as well. Specifically, the sealing unit S3 is the sealing unit 10 that is adjacent to the sealing unit S2 in the circumferential direction, the sealing unit S4 is the sealing unit 10 that is adjacent to the sealing unit S3 in the circumferential direction, the sealing unit S5 is the sealing unit 10 that is adjacent to the sealing unit S4 in the circumferential direction, and the sealing unit S6 is the sealing unit 10 that is adjacent to the sealing unit S5 in the circumferential direction. Accordingly, the sealing units S3 to S6 perform the same operations as the sealing unit S1 at angles that are respectively delayed by 120 degrees, 180 degrees, 240 degrees, and 300 degrees from the sealing unit S1. Accordingly, the operation graphs in the rows for "sealing unit S3", "sealing unit S4", "sealing unit S5", and "sealing unit S6" are respectively shifted 120 degrees, 180 degrees, 240 degrees, and 300 degrees rightward from the operation graph in the row for "sealing unit S1". Note that the sealing units S3 to S6 are likewise configured such that the above-described operations performed by the sealing unit 10 upon reaching the angular positions of about 70 degrees, about 130 degrees, about 140 degrees, about 230 degrees, about 250 degrees, and about 320 degrees.

In this way, the above-described operations are performed in the sequence of sealing unit S1 → sealing unit S2 → sealing unit S3 → sealing unit S4 → sealing unit S5 → sealing unit S6 → and so on. Accordingly, processing for forming the seal portions S is executed in this sequence, and the seal portions S are formed at intervals in the direction of transporting the continuous web 30.

In other words, the formation of seal portions S with intervals therebetween is sequentially executed as the sealing units 10 each rotate along with rotation of the rotation member.

Focusing now on the ultrasonic-wave generation period of the sealing units 10, in the present embodiment, a first period in which an ultrasonic wave generator 9 generates ultrasonic waves for a first sealing unit (which is one of the sealing unit 10; e.g., the sealing unit S1) is overlapped with a second period in which an ultrasonic wave generator 9 generates ultrasonic waves for a second sealing unit (e.g., the sealing unit S2) that is adjacent to the first sealing unit in the circumferential direction.

Specifically, in the case of the start timing and the end timing of the generation of ultrasonic waves for the sealing unit S1 (by the ultrasonic wave generator 9), the generation start timing is about 140 degrees, and the generation end timing is about 230 degrees. However, the sealing unit S2, which is adjacent to the sealing unit S1 in the circumferential direction, is shifted by 60 degrees from the sealing unit S1, and therefore the aforementioned timings are about 200 degrees and about 290 degrees, respectively. In other words, the ultrasonic wave generator 9 for the sealing unit S2 starts to generate ultrasonic waves before the ultrasonic wave generator 9 for the sealing unit S1 ends the generation of ultrasonic waves. Accordingly, the ultrasonic-wave generation period of the sealing unit S1 (first period) and the ultrasonic-wave generation period of the sealing unit S2 (second period) are overlapped with each other.

Also, the ultrasonic-wave generation periods of the five other adjacent pairs of sealing units 10 (i.e., the ultrasonic-wave generation periods of the sealing unit S2 and the sealing unit S3, the ultrasonic-wave generation periods of the sealing unit S3 and the sealing unit S4, the ultrasonic-wave generation periods of the sealing unit S4 and the sealing unit S5, the ultrasonic-wave generation periods of the sealing unit S5 and the sealing unit S6, and the ultrasonic-wave generation periods of the sealing unit S6 and the sealing unit S1) are similarly overlapped with each other.

In other words, for all of the six sealing units 10, the first period in which the ultrasonic wave generator 9 for the first sealing unit generates ultrasonic waves is overlapped with the second period in which the ultrasonic wave generator 9 for the second sealing unit generates ultrasonic waves.

Also, the first period in which the ultrasonic wave generator 9 for the first sealing unit (e.g., the sealing unit S1) generates ultrasonic waves is not overlapped with a third period in which the ultrasonic wave generator 9 for a third sealing unit (e.g., the sealing unit S3), which is adjacent to the second sealing unit (e.g., the sealing unit S2) in the circumferential direction, generates ultrasonic waves.

As previously described, the start timing and the end timing of the generation of ultrasonic waves by the ultrasonic wave generator 9 are about 140 degrees and about 230 degrees for the sealing unit S1, and about 200 degrees and about 290 degrees for the sealing unit S2. Also, the sealing unit S3 that is adjacent to the sealing unit S2 in the circumferential direction is further shifted 60 degrees from the sealing unit S2, and therefore the aforementioned timings are about 260 degrees and about 350 degrees. In other words, the ultrasonic wave generator 9 for the sealing unit S3 starts the generation of ultrasonic waves after the ultrasonic wave generator 9 for the sealing unit S1 ends the generation of ultrasonic waves. Accordingly, the ultrasonic-wave generation period of the sealing unit S1 (first period) and the ultrasonic-wave generation period of the sealing unit S3 (third period) are not overlapped with each other.

Next, the relationship between the repetition cycle of ultrasonic-sealing processing and the ultrasonic-wave generation periods will be described.

As previously described, the sealing units S2 to S6 perform the same operations as the sealing unit S1 at angles that are respectively delayed by 60 degrees, 120 degrees, 180 degrees, 240 degrees, and 300 degrees from the sealing unit S1. In other words, ultrasonic-sealing processing is executed every 60 degrees by one of the six sealing units 10 in order, and therefore letting the angular velocity of the rotation member be V (deg/s), the repetition cycle (cycle time) of the ultrasonic-sealing processing is 60/V (s).

Here, the ultrasonic wave generators 9 for the six sealing units 10 start the generation of ultrasonic waves when the corresponding sealing unit 10 reaches the angular position B1 (about 140 degrees), and end the generation of ultrasonic waves when the angular position B2 (about 230 degrees) is reached. In other words, the ultrasonic wave generators 9 each generate ultrasonic waves for about 90/V (s).

In this way, in the seal-portion forming step of the present embodiment, the ultrasonic wave generators 9 generate ultrasonic waves such that the time from when ultrasonic wave generation starts until when ultrasonic wave generation ends is longer than the repetition cycle of ultrasonic-sealing processing.

### Effectiveness of method for ultrasonic-sealing of present embodiment

As previously described, in the method for ultrasonic-sealing of the present embodiment, in the seal-portion forming step, the ultrasonic wave generators 9 generate ultrasonic waves such that the time from when ultrasonic wave generation starts until when ultrasonic wave generation ends (hereinafter, called the ultrasonic wave generation time) is longer than the repetition cycle (cycle time) of ultrasonic-sealing processing. This therefore makes it possible to suppress a decrease in the quality of the seal portions S.

In order to execute ultrasonic-sealing processing more times and increase the number of absorbent articles produced per unit of time, it is necessary to shorten the repetition cycle of the ultrasonic-sealing processing by increasing the rotation speed of the rotation member, for example. However, if the repetition cycle is shortened, the amount of time for forming the seal portions S is also shortened, and there is a risk of a decrease in the quality of the seal portions S.

To address this, in the present embodiment, in the seal-portion forming step, the ultrasonic wave generators 9 generate ultrasonic waves such that the time from when the generation of ultrasonic waves starts until when the generation of ultrasonic waves ends is longer than the repetition cycle of the ultrasonic-sealing processing. In other words, even if the repetition cycle is shortened in order to increase productivity, the ultrasonic wave generation time is allowed to be longer than the repetition cycle (rather than shortening the ultrasonic wave generation time in accordance with the shortening of the repetition cycle), thus ensuring sufficient time for appropriately forming the seal portions S. This therefore makes it possible to suppress a decrease in the quality of the seal portions S.

Also, in the present embodiment, the continuous web 30 is wound on the outer circumferential surface 5A of the rotation member and transported by rotation of the rotation member, the sealing units 10, each having an ultrasonic horn 8 and an anvil 14, are provided at equal intervals along the circumferential direction of the rotation member, the first members, which are the ultrasonic horns 8 or the anvils 14, are provided on the outer circumferential surface 5A of the rotation member, and the second members, which are the other ones, are provided so as to be able to move toward and away from the first members on the opposite side of the continuous web 30, the sealing units 10 sequentially form seal portions S at intervals while rotating along with the rotation of the rotation member, and a first period in which the ultrasonic wave generator 9 for a first sealing unit out of the sealing units 10 generates ultrasonic waves is overlapped with a second period in which the ultrasonic wave generator 9 for a second sealing unit that is adjacent to the first sealing unit in the circumferential direction generates ultrasonic waves. In other words, even if the repetition cycle is shortened in order to increase productivity, the first period and the second period of adjacent sealing units are allowed to overlap each other, thus ensuring a sufficient length for such periods, that is to say sufficient time for appropriately forming the seal portions S. This therefore makes it possible to suppress a decrease in the quality of the seal portions S.

Furthermore, in the present embodiment, for all of the sealing units 10, the first period in which the ultrasonic wave generator 9 for the first sealing unit generates ultrasonic waves is overlapped with the second period in which the ultrasonic wave generator 9 for the second sealing unit generates ultrasonic waves, thus making it possible to further suppress a decrease in the quality of the seal portions S.

Also, in the present embodiment, the first period in which the ultrasonic wave generator 9 for the first sealing unit generates ultrasonic waves is not overlapped with a third period in which the ultrasonic wave generator 9 for a third sealing unit that is adjacent to the second sealing unit in the circumferential direction generates ultrasonic waves. In other words, three periods of generation of ultrasonic waves by ultrasonic wave generators 9 are not allowed to overlap each other, thus making it possible to suppress a sudden change in the load (power) of the sealing device 1.

### High speed mode and low speed mode

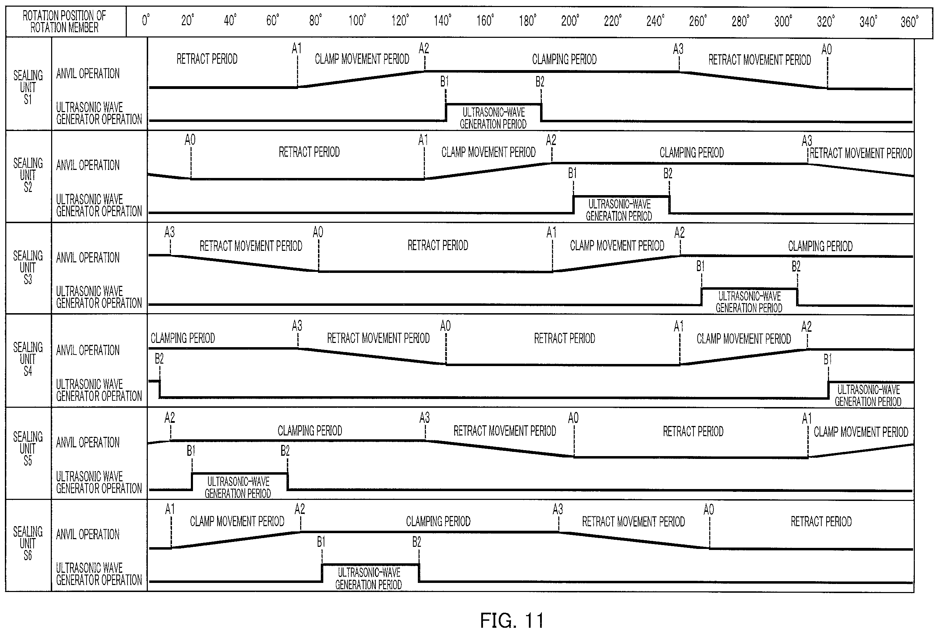

Next, two types of operation modes (i.e., a high speed mode and a low speed mode) will be described with reference to FIGS. 10 and 11. FIG. 11 is an illustrative diagram for illustrating operations of the six sealing units 10 in the low speed mode.

Normally, in order to execute ultrasonic-sealing processing more times per unit of time, the rotation speed of the rotation member is increased when performing ultrasonic-sealing processing. However, there are also cases where the rotation speed of the rotation member is reduced when performing ultrasonic-sealing processing in order to adjust (reduce) the number of times that ultrasonic-sealing processing is executed per unit of time when production adjustment or the like is necessary.

In the present embodiment, ultrasonic-sealing processing is normally executed in the high speed mode, and the operations of the six sealing units 10 in the high speed mode (also called the normal mode) have already be described above with reference to FIG. 10. Accordingly, the following describes operations of the six sealing units 10 in the low speed mode (also called the adjustment mode) with reference to FIG. 11.

Similarly to FIG. 10, in FIG. 11 as well, the sealing units 10 each perform the above-described corresponding operations upon reaching the above-described angular positions A0, A1, A2, A3, B1, and B2. Also, likewise to the previous description, the sealing units S2 to S6 perform the same operations as the sealing unit S1 at delays of 60 degrees, 120 degrees, 180 degrees, 240 degrees, and 300 degrees respectively from the sealing unit S1.

In the low speed mode shown in FIG. 11, the angular positions of the sealing unit S1 (which match the rotation positions of the rotation member similarly to the above description in the present embodiment) are the angular position A1 of about 70 degrees, the angular position A2 of about 130 degrees, the angular position B1 of about 140 degrees, the angular position B2 of about 185 degrees, the angular position A3 of about 250 degrees, and the angular position A0 of about 320 degrees. The angular positions A1, A2, B1, A3, and A0 are the same as the angular positions of the sealing unit S1 in the high speed mode shown in FIG. 10, but B2 is different. Specifically, whereas B2 is about 230 degrees in the high speed mode shown in FIG. 10, B2 is about 185 degrees in the low speed mode shown in FIG. 11.

Accordingly, in the high speed mode (normal mode) , the first period in which the ultrasonic wave generator 9 generates ultrasonic waves for the first sealing unit is overlapped with the second period in which the ultrasonic wave generator 9 generates ultrasonic waves for the second sealing unit that is adjacent to the first sealing unit in the circumferential direction. But in the low speed mode (adjustment mode), the first period and the second period are not overlapped with each other.

Specifically, in the low speed mode (first speed), the period from the start to the end of the generation of ultrasonic waves by the ultrasonic wave generator 9 for the sealing unit S1 is from about 140 degrees to about 185 degrees. The period for the sealing unit S2 that is adjacent to the sealing unit S1 in the circumferential direction is about 200 degrees to about 245 degrees. In other words, the ultrasonic wave generator 9 for the sealing unit S2 starts to generate ultrasonic waves after the ultrasonic wave generator 9 for the sealing unit S1 ends the generation of ultrasonic waves. Accordingly, in the high speed mode (second speed), the first period and the second period are overlapped with each other as previously described, but in the low speed mode, the ultrasonic-wave generation period of the sealing unit S1 (first period) and the ultrasonic-wave generation period of the sealing unit S2 (second period) are not overlapped with each other.

As previously described, when the rotation speed of the rotation member is high (i.e., when in the high speed mode), the ultrasonic-wave generation periods need to be overlapped with each other in order to ensure sufficient time for appropriately forming the seal portions S. However, in the low speed mode, even when sufficient time is ensured, the range of angles over which the rotation member rotates over such time (in other words, the ultrasonic-wave generation periods in FIG. 11) is smaller than in the high speed mode, and therefore there is no need for the periods to overlap each other.

In the present embodiment, whether or not the ultrasonic-wave generation periods of the six sealing units 10 overlap each other is changed according to the operation mode. Accordingly, in both of the modes, it is possible to ensure sufficient time for appropriately forming the seal portions S, and it is possible to suppress a decrease in the quality of the seal portions S.

Also, in the present embodiment, for each of the sealing units 10, when the sealing unit 10 reaches the predetermined angular position B1 while rotating along with rotation of the rotation member, the ultrasonic wave generator 9 starts to generate ultrasonic waves.

For this reason, regardless of whether the rotation speed of the rotation member is fast or is slow (the repetition cycle of ultrasonic-sealing processing is long), the ultrasonic wave generator 9 starts to generate ultrasonic waves when the sealing unit 10 reaches the predetermined angular position B1. Accordingly, regardless of the rotation speed of the rotation member, ultrasonic waves can be reliably generated at a desired timing (e.g., can be reliably started in the clamping state after clamping is performed at the angular position A2).

### Second Embodiment

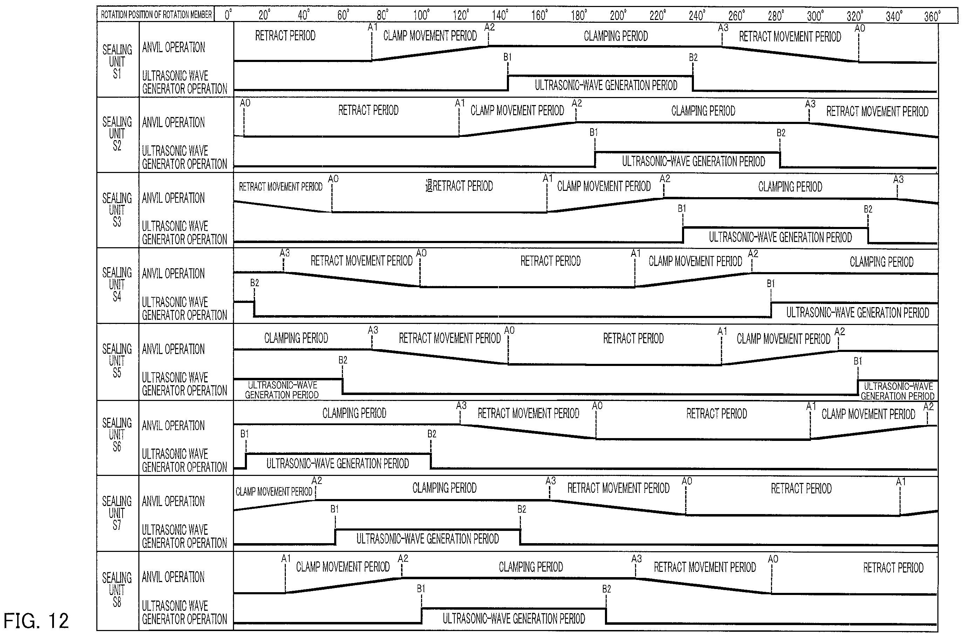

FIG. 12 is an illustrative diagram that corresponds to FIG. 10 and is for illustrating operations of sealing units 10 according to a second embodiment.

The sealing device 1 of the second embodiment has eight sealing units 10 arranged at intervals of 45 degrees along the circumferential direction. Hereinafter, the sealing units 10 will be called a sealing unit S1, a sealing unit S2, a sealing unit S3, a sealing unit S4, a sealing unit S5, a sealing unit S6, a sealing unit S7, and a sealing unit S8, in order along the circumferential direction.

Similarly to the first embodiment (FIG. 10), in the second embodiment (FIG. 12) as well, upon reaching the angular positions A0, A1, A2, A3, B1, and B2, the sealing units 10 each perform an operation that corresponds to the angular position. In the second embodiment, the angular positions of the sealing unit S1 (which match the rotation positions of the rotation member similarly to the first embodiment) are the angular position A1 of about 70 degrees, the angular position A2 of about 130 degrees, the angular position B1 of about 140 degrees, the angular position B2 of about 235 degrees, the angular position A3 of about 250 degrees, and the angular position A0 of about 320 degrees.

Also, the sealing unit S2 is the sealing unit 10 that is adjacent to the sealing unit S1 in the circumferential direction, and is separated by an angle of 45 degrees from the sealing unit S1. Accordingly, the sealing unit S2 performs the same operations as the sealing unit S1, at a delay of 45 degrees from the sealing unit S1. Accordingly, the operation graph in the row for "sealing unit S2" corresponds to a 45-degree shift rightward from the operation graph in the row for "sealing unit S1",and the above-described operations are executed when the rotation position of the rotation member reaches angles obtained by adding 45 degrees to the angles for the sealing unit S1, that is to say about 115 degrees, about 175 degrees, about 185 degrees, about 280 degrees, about 295 degrees, and about 5 degrees.

Hereinafter, similarly to the first embodiment, operations are similarly executed in the case of the sealing units S3 to S8 as well. In other words, the sealing units S2 to S8 perform the same operations as the sealing unit S1 at delays of 45 degrees, 90 degrees, 135 degrees, 180 degrees, 225 degrees, 270 degrees, and 315 degrees respectively from the sealing unit S1.

As previously described, in the second embodiment, the period from the start to the end of the generation of ultrasonic waves is from about 140 degrees to about 235 degrees for the ultrasonic wave generator 9 for the sealing unit S1, and from about 175 degrees to about 280 degrees for the ultrasonic wave generator 9 for the sealing unit S2. Also, the sealing unit S3 that is adjacent to the sealing unit S2 in the circumferential direction is further shifted 45 degrees from the sealing unit S2, and therefore the aforementioned period is from about 230 degrees to about 325 degrees. In other words, the ultrasonic wave generator 9 for the sealing unit S3 starts to generate ultrasonic waves before the ultrasonic wave generator 9 for the sealing unit S1 ends the generation of ultrasonic waves.

In other words, three or more (here, three) periods of the generation of ultrasonic waves by the ultrasonic wave generators 9 for three or more (here, three) consecutively adjacent sealing units 10 have a shared overlapping portion (the period from about 230 degrees to about 235 degrees). Note that although three periods have a shared overlapping portion in the example in FIG. 12, in other examples (e.g., when there are even more sealing units 10 and the about-described shift angle is therefore even smaller), there can be cases where four or more periods have an overlapping portion with each other.

The repetition cycle of ultrasonic-sealing processing in the second embodiment is 45/V (s), which is shorter than in the case of the first embodiment. In this case as well, if the shared overlapping portion is provided, it is possible to ensure sufficient time for appropriately forming the seal portions S, and it is possible to suppress a decrease in the quality of the seal portions S.

Also, in the second embodiment, the angular position at which the sealing unit S1 clamps the continuous web 30 is about 130 degrees, and the angular position A3 at which the continuous web is unclamped is 250 degrees. In the case of the three consecutively adjacent sealing units 10 (ultrasonic wave generators 9) beginning from the sealing unit S1, the timing of the start of the generation of ultrasonic waves is about 140 degrees for the sealing unit S1, about 185 degrees for the sealing unit S2, and about 230 degrees for the sealing unit S3.

Specifically, in the period from when the first sealing unit (e.g., the sealing unit S1) starts to clamp the continuous web 30 until when it unclamps the continuous web 30, the corresponding ultrasonic wave generators 9 start to generate ultrasonic waves for the first sealing unit, the second sealing unit (e.g., the sealing unit S2) that is adjacent to the first sealing unit in the circumferential direction, and the third sealing unit (e.g., the sealing unit S3) that is adjacent to the second sealing unit in the circumferential direction. In other words, the ultrasonic wave generators 9 for the sealing units S1 to S3 start to generate ultrasonic waves in the period from when the sealing unit S1 starts to clamp the continuous web 30 until when it unclamps the continuous web 30.

As previously described, the repetition cycle of ultrasonic-sealing processing is shorter in the second embodiment than in the first embodiment. For this reason, the ultrasonic wave generators 9 for the first to third sealing units start to generate ultrasonic waves while the first sealing unit is clamping the continuous web, thus making it possible to ensure sufficient time for the ultrasonic wave generators 9 to generate ultrasonic waves in the ultrasonic-sealing processing (i.e., sufficient time for appropriately forming the seal portions S) , and making it possible to suppress a decrease in the quality of the seal portions S.

### Third Embodiment

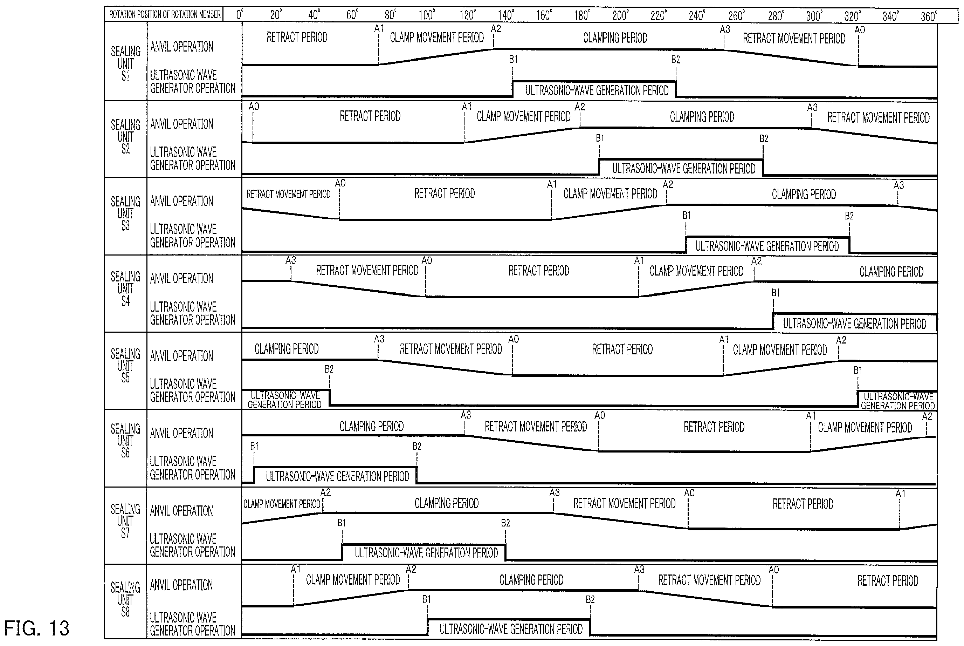

FIG. 13 is an illustrative diagram that corresponds to FIG. 10 and is for illustrating operations of sealing units 10 according to a third embodiment.

The third embodiment and the second embodiment are different in terms of the angular position B2. Specifically, similarly to the second embodiment, the sealing device 1 of the third embodiment has eight sealing units 10 arranged at intervals of 45 degrees along the circumferential direction, and the sealing units S2 to S8 perform the same operations as the sealing unit S1 at delays of 45 degrees, 90 degrees, 135 degrees, 180 degrees, 225 degrees, 270 degrees, and 315 degrees respectively from the sealing unit S1.

In the third embodiment, the angular positions of the sealing unit S1 (which match the rotation positions of the rotation member) are the angular position A1 of about 70 degrees, the angular position A2 of about 130 degrees, the angular position B1 of about 140 degrees, the angular position B2 of about 225, the angular position A3 of about 250 degrees, and the angular position A0 of about 320 degrees. As previously mentioned, B2 is different from the angular positions of the sealing unit S1 of the second embodiment shown in FIG. 12. Specifically, B2 is about 235 degrees in the second embodiment, but is about 225 degrees in the third embodiment. The reason for this is that, because the rotation speed of the rotation member in the third embodiment is slower than the rotation speed of the rotation member in the second embodiment, even if the previously described predetermined energy amount is the same and the ultrasonic wave generation time is the same, the range of angles over which the rotation member rotates over such time (in other words, the ultrasonic-wave generation period in FIG. 13) is smaller than in the second embodiment.

Also, in the third embodiment, the ultrasonic-wave generation period is from about 140 degrees to about 225 degrees for the sealing unit S1, about 175 degrees to about 270 degrees for the sealing unit S2, and about 230 degrees to about 315 degrees for the sealing unit S3. In other words, the ultrasonic wave generator 9 for the sealing unit S3 starts to generate ultrasonic waves after the ultrasonic wave generator 9 for the sealing unit S1 ends the generation of ultrasonic waves.

In the second embodiment, three or more periods of the generation of ultrasonic waves by the ultrasonic wave generators 9 for three or more consecutively adj acent sealing units 10 have a shared overlapping portion, but in the third embodiment, the first period in which the ultrasonic wave generator 9 for the first sealing unit generates ultrasonic waves is not overlapped with the third period in which the ultrasonic wave generator 9 for the third sealing unit, which is circumferentially adjacent to the second sealing unit that is adjacent to the first sealing unit in the circumferential direction, generates ultrasonic waves.

Similarly to the second embodiment, in the third embodiment, the ultrasonic wave generators 9 for the first to third sealing units start to generate ultrasonic waves in the period from when the first sealing unit starts to clamp the continuous web 30 until when it unclamps the continuous web 30. For this reason, even if the repetition cycle of ultrasonic-sealing processing is set even shorter than in the first embodiment, it is possible to ensure sufficient time for the ultrasonic wave generators 9 to generate ultrasonic waves in the ultrasonic-sealing processing (i.e., sufficient time for appropriately forming the seal portions S), and it is possible to suppress a decrease in the quality of the seal portions S.

Also, the first period in which the ultrasonic wave generator 9 for the first sealing unit generates ultrasonic waves is overlapped with the second period in which the ultrasonic wave generator 9 for the second sealing unit generates ultrasonic waves, but the first period is not overlapped with the third period in which the ultrasonic wave generator 9 for the third sealing unit generates ultrasonic waves. Also, the second period and the third period are overlapped with each other. In other words, the periods of ultrasonic wave generation by ultrasonic wave generators 9 that are overlapped with each other are two periods of adjacent sealing units 10. In other words, in the third embodiment, three periods of generation of ultrasonic waves by ultrasonic wave generators 9 are not overlapped with each other, thus making it possible to suppress a sudden change in the load (power) of the sealing device 1.

### Other embodiments

Although the embodiments of the present disclosure have been described hereinabove, the above embodiments of the present disclosure are simply to facilitate understanding of the present disclosure and are not in any way to be construed as limiting the present disclosure. The present disclosure may variously be changed or altered without departing from its gist and encompass equivalents thereof.

In the above embodiments, the ultrasonic wave generator 9 that generates ultrasonic waves for the first sealing unit (e.g., the sealing unit S1) and the ultrasonic wave generator 9 that generates ultrasonic waves for the second sealing unit (e.g., the sealing unit S2) are different ultrasonic wave generators 9. In other words, one ultrasonic wave generator 9 is provided for each of the sealing units 10, but there is no limitation to this. For example, instead of the ultrasonic wave generators 9 and the sealing units 10 being provided in one-to-one correspondence, one ultrasonic wave generator 9 may be provided in correspondence with multiple sealing units 10.

In the case of providing one ultrasonic wave generator 9 for each of the sealing units 10, one ultrasonic wave generator 9 is not required to generate ultrasonic waves for multiple sealing units 10. In other words, the ultrasonic wave generator 9 does not need to perform reading/writing/switching a memory for each one of multiple corresponding sealing units 10 (each time the corresponding sealing unit 10 changes) . Also, time is not required for such reading/writing/switching, and the ultrasonic wave generator 9 need only generate ultrasonic waves for one sealing unit 10, and therefore even if the above-described operation mode is the high speed mode, ultrasonic wave generation can be performed without any problem (with sufficient allowance). The above embodiment is desirable in this respect.

Also, in the above embodiment, when the processing for forming a seal portion S is executed, the ultrasonic wave generator 9 starts to generate ultrasonic waves, and then the ultrasonic wave generator 9 stops generating ultrasonic waves when the ultrasonic vibration energy (electric energy) reaches a predetermined amount, but there is no limitation to this. For example, the generation of ultrasonic waves may be stopped after a certain time has elapsed since the ultrasonic wave generator 9 started to generate ultrasonic waves, or when the instantaneous value of the ultrasonic vibration energy of the ultrasonic waves generated by the ultrasonic wave generator 9 (corresponding to the power of the ultrasonic wave generator 9) has exceeded a predetermined threshold value.

Also, in the above embodiments, out of the ultrasonic horns 8 and the anvils 14, the first members are provided on the outer circumferential surface 5A of the rotation member (rotating drum 5), and the second members are provided on the opposite side of the continuous web 30 so as to be able to move toward and away from the first members (ultrasonic horns 8), and the first members are the ultrasonic horns 8 and the second members are the anvils 14. However, there is no limitation to this, and a configuration is possible in which the first members are the anvils 14 and the second members are the ultrasonic horns 8.

Also, although a link mechanism that employs a cam is described as the swing drive portion in the above embodiment, there is no limitation to this. The operations of the ultrasonic horn 8 and the anvil 14 in the above embodiment may be realized with use of a cylinder mechanism or the like instead of a link mechanism that employs a cam.

Also, although an air damper is used as the first elastic member 71 and the second elastic member 72 in the above embodiment, there is no limitation to this. A coil spring or the like may be used instead of an air damper.

### [Reference Signs List]

1 sealing device, 2 timing wheel, 3 bearing portion, 3a rotation shaft
3b ball bearing, 4 fixed table, 5 rotating drum, 5A outer circumferential surface
5a rotating drum window, 6 rotation base, 7a converter, 7b booster
8 ultrasonic horn, 8a horn facing surface, 9 ultrasonic wave generator, 10 sealing unit
14 anvil, 14B anvil projection portion, 14a anvil facing surface
21 supply transporting roll, 22 discharge transporting roll, 30 continuous web
30A one side of continuous web, 30B other side of continuous web, 31 second sheet
32 first sheet, 32a side edge on one side of first sheet
32b side edge on other side of first sheet, 33 liquid-absorbent body, 34 leg hole
35 first waist band, 36 second waist band, 37 first leg band
38 second leg band, 50 swinging support member, 51 swing portion, 51A swing shaft
51B first coupling shaft, 52 holding portion, 52B attachment surface, 52a frame portion, 52c inward surface
53 drive link, 54 rotation link, 54A rotation link base portion
54B rotation link arm portion, 54a support shaft, 55 second coupling shaft, 56 drive member
56a drive support body, 56b follower, 60 cam member, 61 cam groove
70 support portion, 71 first elastic member, 71A first fixing plate, 71B second fixing plate
72 second elastic member, 73 first nozzle, 74 second nozzle, 75 first air pipe
76 second air pipe, S seal portion

## Claims

1. An method for ultrasonic-sealing in which a plurality of seal portions are formed in a continuous web at an interval in a direction of transporting the continuous web,
the forming being performed by repeatedly executing ultrasonic-sealing processing on a continuous web that is being transported,
the continuous web being for an absorbent article,
the ultrasonic-sealing processing comprising:
a clamping step of clamping the continuous web with an ultrasonic horn and an anvil;
a seal-portion forming step of forming the seal portions in the continuous web by vibration of the ultrasonic horn due to receiving an ultrasonic wave generated by an ultrasonic wave generator; and
an unclamping step of unclamping the continuous web,
in the seal-portion forming step,
the ultrasonic wave generator generating the ultrasonic wave such that a period of time from when the generation of the ultrasonic wave starts until when the generation of the ultrasonic wave ends is longer than a repetition cycle of the ultrasonic-sealing processing.

2. The method for ultrasonic-sealing according to claim 1, wherein
the continuous web is transported by rotation of the rotation member with being wound on an outer circumferential surface of a rotation member,
a plurality of sealing units that each have an ultrasonic horn and an anvil are provided at an equal spacing along a circumferential direction of the rotation member,
a plurality of first members, which are either of the ultrasonic horns and the anvils, are provided on the outer circumferential surface of the rotation member,
a plurality of second members, which are other ones out of the ultrasonic horns and the anvils, are each provided on an opposite side of the continuous web so as to be capable of moving toward and away from the corresponding first member,
the plurality of sealing units sequentially form the plurality of seal portions at the interval while rotating along with rotation of the rotation member, and
out of the plurality of sealing units, concerning a first sealing unit and a second sealing unit that is adjacent to the first sealing unit in the circumferential direction,
letting a first period to be a period in which the ultrasonic wave generator for the first sealing unit generates the ultrasonic wave, and a second period to be a period in which the ultrasonic wave generator for the second sealing unit generates the ultrasonic wave, the first period is overlapped with the second period.

3. The method for ultrasonic-sealing according to claim 2, wherein
the ultrasonic wave generator that generates the ultrasonic wave for the first sealing unit and the ultrasonic wave generator that generates the ultrasonic wave for the second sealing unit are different from each other.

4. The method for ultrasonic-sealing according to claim 2 or 3, wherein
for each of the sealing units, when the sealing unit reaches a predetermined angular position while rotating along with rotation of the rotation member, the ultrasonic wave generator starts to generate the ultrasonic wave.

5. The method for ultrasonic-sealing according to any of claims 2 to 4, wherein
concerning the first period which is a period in which the ultrasonic wave generator for the first sealing unit generates the ultrasonic wave, and the second period which is a period in which the ultrasonic wave generator for the second sealing unit generates the ultrasonic wave,
for all of the sealing units, the first period is overlapped with the second period.

6. The method for ultrasonic-sealing according to any of claims 2 to 5, wherein
concerning the first period which is a period in which the ultrasonic wave generator for the first sealing unit generates the ultrasonic wave, and a third period which is a period in which the ultrasonic wave generator for a third sealing unit that is adjacent to the second sealing unit in the circumferential direction generates the ultrasonic wave,
the first period is not overlapped with the second period.

7. The method for ultrasonic-sealing according to any of claims 2 to 5, wherein
three or more periods of generation of the ultrasonic wave by the ultrasonic wave generator for three or more consecutively adj acent sealing units have a shared overlapping portion.

8. The method for ultrasonic-sealing according to claim 2, wherein
when the first sealing unit reaches a predetermined clamp angular position while rotating along with rotation of the rotation member,
the second member comes into contact with the corresponding first member and clamps the continuous web,
when the first sealing unit reaches a predetermined unclamp angular position while rotating along with rotation of the rotation member,
the second member moves away from the first member and unclamps the continuous web, and
in a period from when the first sealing unit starts to clamp the continuous web until when the first sealing unit unclamps the continuous web,
the ultrasonic wave generator start to generate the ultrasonic wave for the first sealing unit, the second sealing unit, and a third sealing unit that is adjacent to the second sealing unit in the circumferential direction.

9. The method for ultrasonic-sealing according to claim 8, wherein
concerning the first period which is a period in which the ultrasonic wave generator for the first sealing unit generates the ultrasonic wave, and a third period which is a period in which the ultrasonic wave generator for a third sealing unit that is adjacent to the second sealing unit in the circumferential direction generates the ultrasonic wave,
the first period is not overlapped with the second period.

10. The method for ultrasonic-sealing according to claim 1, wherein
the continuous web is transported by rotation of the rotation member with being wound on an outer circumferential surface of a rotation member,
a plurality of sealing units that each have an ultrasonic horn and an anvil are provided at an equal interval along a circumferential direction of the rotation member,
a plurality of first members, which are either of the ultrasonic horns and the anvils, are provided on the outer circumferential surface of the rotation member,
a plurality of second members, which are other ones out of the ultrasonic horns and the anvils, are each provided on an opposite side of the continuous web so as to be capable of moving toward and away from the corresponding first member,
the plurality of sealing units sequentially form the plurality of seal portions at the interval while rotating along with rotation of the rotation member,
the plurality of seal portions are formed in a low speed mode and in a high speed mode,
the low speed mode being a mode in which the continuous web is transported by rotation of the rotation member at a first speed,
the high speed mode being a mode in which the continuous web is transported by rotation of the rotation member at a second speed that is higher than the first speed,
out of the plurality of sealing units, concerning a first sealing unit and a second sealing unit that is adjacent to the first sealing unit in the circumferential direction,
letting a first period to be a period in which the ultrasonic wave generator for the first sealing unit generates the ultrasonic wave, and a second period to be a period in which the ultrasonic wave generator for the second sealing unit generates the ultrasonic wave,
in the high speed mode, the first period is overlapped with the second period, and
in the low speed mode, the first period and the second period are not overlapped with each other.

11. An device for ultrasonic-sealing that forms a plurality of seal portions in a continuous web that is for an absorbent article,
the device for ultrasonic-sealing comprising:
a transporting device configured to transport the continuous web;
an ultrasonic wave generator configured to generate an ultrasonic wave;
an ultrasonic horn configured to form the seal portion in the continuous web that is being transported by the transporting device, by vibrating due to receiving the ultrasonic wave generated by the ultrasonic wave generator; and
an anvil configured to clamp the continuous web together with the ultrasonic horn,
the ultrasonic horn forming the plurality of seal portions at an interval in a direction of transporting the continuous web by repeatedly executing ultrasonic-sealing processing,
the ultrasonic-sealing processing being for forming a seal portion in the continuous web by vibrating while clamping the continuous web together with the anvil,
the ultrasonic wave generator generating the ultrasonic wave such that a period of time from when the generation of the ultrasonic wave starts until when the generation of the ultrasonic wave ends is longer than a repetition cycle of the ultrasonic-sealing processing.
